# FASCICULE DE BREVET EUROPEEN

(11) **EP 1 499 304 B1**
(45) Date de publication et mention de la délivrance du brevet: **07.03.2018**
(21) Numéro de dépôt: 03740610.5
(22) Date de dépôt: 15.04.2003
(51) Int. Cl.: A61K 9/48

(54) **COMPOSITIONS LIQUIDES POUR CAPSULES MOLLES A LIBERATION PROLONGEE ET LEUR PROCEDE DE FABRICATION**
FLÜSSIGE ZUSAMMENSETZUNGEN MIT VERLÄNGERTER FREISETZUNG FÜR WEICHKAPSELN UND HERSTELLUNGSVERFAHREN
LIQUID COMPOSITIONS FOR SOFT SUSTAINED-RELEASE CAPSULES AND METHOD FOR PRODUCTION THEREOF

(30) Priorité: 15.04.2002 FR 0204697
(43) Date de publication de la demande: 26.01.2005
(73) Titulaire: Paris, Laurence, 03600 Commentry (FR)
(72) Inventeur: Paris, Laurence, 03600 Commentry (FR)
(74) Mandataire: Herrero Herranz, Eva
(86) Numéro de dépôt international: PCT/FR2003/001195
(87) Numéro de publication internationale: WO 2003/086368

(56) Documents cités:
- EP-A- 0 095 123
- EP-A- 0 173 293
- WO-A-01/35922
- WO-A-99/36445
- WO-A-99/42521
- FR-A- 2 774 907
- US-A1- 2001 051 686
- US-A1- 2001 053 801
- US-A1- 2002 032 243
- PATENT ABSTRACTS OF JAPAN vol. 013, no. 049 (C-565), 3 février 1989 (1989-02-03) & JP 63 246322 A (TOYO KAPUSERU KK), 13 octobre 1988 (1988-10-13)
- DATABASE WPI Section Ch, Week 198847 Derwent Publications Ltd., London, GB; Class B05, AN 1988-333837 XP002223635 & JP 63 246333 A (TOYO CAPSULE KK), 13 octobre 1988 (1988-10-13)

## Description

### DOMAINE D'APPLICATION DE L'INVENTION

La présente invention a trait au domaine pharmaceutique, cosmétique et diététique et plus particulièrement à celui des systèmes à libération prolongée des principes actifs dans le corps humain.

### DESCRIPTION DE L'ART ANTERIEUR

Parmi les voies d'administration des principes actifs dans le corps humain, la voie orale représente la voie de prédilection pour l'administration de systèmes à libération prolongée. La majorité de ces systèmes se présentent sous forme solide. Ce sont les comprimés et certaines gélules contenant des microgranules. Ces formes à libération prolongée ou dites programmées sont nombreuses et appartiennent à différentes catégories en fonction des excipients utilisés pour ralentir la libération des principes actifs. Ce sont :
- les formes matricielles :
   * les matrices hydrophiles à base de dérivés de cellulose, de dérivés d'amidon et autres polysaccharides ayant des propriétés de gonflement entraînant la formation d'une gangue visqueuse au contact des sucs digestifs. La libération du principe actif dans le cas présent est fonction :
      ∘ de l'épaisseur de la gangue visqueuse
      ∘ des enzymes présentes dans le milieu digestif. Dans le cas des matrices hydrophiles les amylases sont très actives sur les cellulose et les amidons
      ∘ et d'un phénomène d'érosion.
   * les matrices inertes à base de matières plastiques tel que le PVC, les résines métacryliques ("Eudragit®)", les carbomers ("Carbopol®)", etc....... La libération du principe actif dans le cas présent se fait par :
      ∘ un simple mécanisme de solubilisation/diffusion à travers des canalicules
      ∘ et par un phénomène d'érosion progressif de la matrice.
   * les matrices minérales à base de phosphates de calcium, etc...... Le mécanisme de libération du principe actif est identique à celui des matrices inertes.
   * les matrices lipidiques à base de glycérides (mono -, di - et tri -glycérides), des acides et alcools gras, divers esters d'acides gras et d'alcools de bas poids moléculaire, des cires constituées principalement d'esters d'alcools et d'acides supérieurs, etc...... La libération du principe actif dans le cas présent est fonction :
      ∘ du point de fusion de la masse grasse
      ∘ de la balance hydro -lipophile (HLB) de la masse grasse
      ∘ des enzymes digestives telles que des lipases présentes dans le suc pancréatique
      ∘ et du phénomène d'érosion de la matrice. Le point de fusion de la masse, son HLB et l'action des enzymes digestives font que la libération du principe actif à partir d'une telle matrice est très difficile à maîtriser in vitro mais aussi in vivo car la température du corps humain ainsi que la production des enzymes digestives conduisent à des variations intra et inter individu très importantes.
   * Les micro -matrices ou pellets à base des différents constituants cités précédemment. La libération du principe actif est régie de la même façon que dans les différents cas précédents
- les formes pelliculées. Ce sont des comprimés classiques et des microgranules ayant subit un enrobage à l'aide de substances ayant des propriétés particulières permettant une libération lente à travers la membrane formée. La libération peut être soit :
   * pH dépendante. Le film se dissout progressivement en fonction du pH du tube digestif. Généralement ces substances sont très peu solubles en milieu acide et le deviennent progressivement en fonction du pH du tube digestif allant de 1.2 (estomac) à 5.3 (intestin proximal), 6.8 (intestin distal) et 7.5 (gros intestin). Ces films sont généralement constitués de résines métacryliques solubles à différents pH.
   * pH indépendant. Le film ne se dissout pas mais devient progressivement poreux en fonction du pH du tube digestif. Ces films à base de résines métacryliques sont idéaux car aucun facteur biologique n'intervient sur leurs propriétés mécaniques. Il se forme ainsi des membranes osmotiques à travers lesquelles diffuse le principe actif dissous.

La deuxième forme d'administration de principes actifs par voie orale, est la forme liquide. Elle se présente sous forme :
- de solutions aqueuses ou hydro -alcooliques ou contenant des solvants comme des polyoxyéthylène glycols, du propylène glycol, etc......
- des suspensions aqueuses ou hydro -alcooliques ou contenant d'autres solvants acceptés sur le plan toxicologique.

Ces formes peuvent être présentées en « vrac », flacons, ou en dose unitaire telles que les capsules molles ou les gélules "Licaps®" à contenu liquide. Sous cette forme, très peu de formes à libération prolongée existent en raison de la difficulté à éviter la libération du principe actif in situ au cours du temps.

Des essais ont été réalisés en fixant les principes actifs sur des résines échangeuses d'ions, tels que les résinates de codéine, de pholcodine, de phényltoloxamine, etc......

Les brevets Américain US 3244588 et Anglais GB 10056458 de MPHILLER Nielsen font mention de la réalisation de ces résines échangeuses d'ions pour les antitussifs. Ces complexes peuvent être ainsi dispersés dans une phase ne permettant pas la libération du principe
actif et présenté soit sous forme « vrac » ou sous forme de capsules molles ou gélules.

Un autre brevet européen EP0173293 de MERELL DOW fait mention de la réalisation d'une matrice lipidique à base de paraffine solide et de polyéthylène glycol pour un conditionnement en capsules molles.

Les brevets US 5776482 et WO9501787 font part d'un système à libération prolongée présenté sous forme de microgranules enrobés et dispersées dans une phase huileuse classique. Ces microgranules ont la particularité d'être enrobées en vue de libérer progressivement dans le temps le principe actif après ouverture de la capsule et dispersion de ces dernières dans les sucs digestifs.

Le Brevet US 5645848 présente un système à libération prolongée sous forme de capsules molles pour le nettoyage des lentilles. La libération progressive des constituants est obtenue par attaque enzymatique de la tunique de gélatine présentant une composition particulière.

D'autre part, tout un travail a été réalisé par NASHED Norman, thèse d'Université Louis Pasteur, Strasbourg, 1984 -1985, concernant les capsules molles à libération prolongée, reprenant les travaux sur les matrices lipidiques ("Witepsol®", "Gelucire®", "Suppocire®", "Precirol®"), sur certains dérivés naturels présentant un phénomène de précipitation in situ au contact de l'eau (gomme laque et colophane), et sur certains polymères tels que les silicones. Dans tous les cas, les résultats se sont avérés négatifs en raison d'une libération soit trop rapide soit trop lente difficilement maîtrisable.

La libération *in vivo* trop rapide a été observée dans le cas des matrices lipidiques et ceci malgré des résultats tout à fait convenables *in vitro.* Cette différence provient du fait de l'action des enzymes digestives, non prise en compte lors des essais *in vitro.*

Les seuls résultats positifs ont été observés sur des capsules molles ayant un an d'ancienneté. Une étude comparative avec les essais *in vivo* à T = 0, montre que ce type de capsules molles vieillit mal. En effet les matières grasses utilisées pour la réalisation de ce type de capsules présentent un phénomène de polymorphisme jouant un rôle prépondérant sur le point de fusion de la masse grasse. Ainsi, un échauffement de la masse grasse peut entraîner un changement non négligeable de la biodisponibilité du principe actif à partir d'une telle matrice. Ceci a été largement étudié par MOES (A) (Pharma. Acta Helv., 1980, 55, 307 -311) et (Sci. Techn. Pharm., 1980, 9, 263 -288), LUTTON (E.S) (J. Am. Oil Chem. Soc., 1972, 49, 1) et BOYMOND C. et HANS J. B. (Bulletin de la Société de Pharmacie, Strasbourg, 1978, 22, 203 -217).

Le document GB1242547 décrit une capsule remplie d'un mélange liquide ou fluide. Dès que le contenu de la capsule vient au contact des sucs digestifs, il se forme une matrice microporeuse, comparable à une mousse. Les agents formateurs de mousse sont soit des matières d'origine naturelle (comme l'éthylcellulose) soit des polymères produits par synthèse comme par exemple le polyvinylacetate.

Une étude approfondie de tous les brevets dans le domaine de la capsule molle n'a pas permis de mettre en évidence une solution efficace pour l'obtention d'une capsule molle à libération prolongée.

### DESCRIPTION DE L'INVENTION

Partant de cet état de fait et pour y remédier, l'invention propose un concept original de compositions liquides selon la revendication 1. Ces compositions sont destinées à la réalisation de capsules à libération prolongée, remarquables en ce que la libération prolongée du principe actif est obtenue par formation *in situ* d'une matrice qui, plus ou moins compacte et biodégradable, est obtenue par modification physique instantanée du contenu de la capsule au contact des sucs digestifs dès son ouverture conduisant à une libération sur une période supérieure à une heure du principe actif préalablement dissous ou dispersé à l'aide de solvants, cette libération étant modulable par incorporation d'adjuvants appropriés.

La présente invention a ainsi pour but de réaliser in situ, après ouverture de la capsule molle ou de la gélule dure "Licaps®", une matrice biodégradable ou non, à partir de laquelle la libération du principe actif est dans la mesure du possible pH indépendant et/ou dépendant de l'action des sucs digestifs en fonction des excipients utilisés pour réaliser ou pour renforcer la solidité de la dite matrice. La réalisation de la matrice in situ est telle que celle-ci est quasi instantanée dès ouverture de la capsule voire initiée au sein de la capsule molle ou gélule "Licaps®".

On entend par «biodégradable» la dégradation d'un support engendrée par un mécanisme biologique tel que l'action des enzymes mais aussi un mécanisme d'érosion mécanique dû au péristaltisme intestinal.

On entend par « initiée », l'apparition d'une ébauche de matrice au sein de la capsule avant ouverture ou dissolution de la tunique de cette dernière.

Cette invention est applicable aussi bien aux capsules molles et gélules dures en gélatine mais aussi aux capsules molles ou gélules dures avec une tunique réalisée avec d'autres matériaux autres que la gélatine tels que les carraghénanes, les amidons et leur dérivés, les hydroxypropylméthylcelluloses et leurs dérives ainsi que les polymères de l'alcool polyvinylique.

Cette invention est basée sur le fait que certaines substances à l'état liquide dans des solvants non toxiques pour l'organisme humain, se gélifient ou forment un réseau poreux, très rapidement au contact de l'eau ou des sucs digestifs. Ainsi il y a formation d'une gangue visqueuse ou d'une structure spongieuse à partir de laquelle le principe actif diffuse progressivement dans le temps. Ces substances étant en majorité des matières synthétiques très utilisées dans le domaine pharmaceutique et cosmétique, la libération du principe actif est peu pH dépendant mais surtout n'est pas ou peu influencée par les enzymes digestives du fait de la protection apportée par la gangue visqueuse vis-à -vis des additifs pouvant être ajoutés pour moduler la libération du principe actif au
cours du temps.

On entend par «gélifier» au sens large, soit l'épaississement d'une masse liquide soit l'obtention d'une masse solide souple tel que cela est observé avec la gélatine.

On entend préférentiellement par «rapidement», la modification instantanée du contenu après ouverture de la capsule, dans un laps de temps compris entre la seconde et 10 minutes.

L'obtention des dites matrices faisant l'objet de la présente invention fait appel à des substances dites matricielles qui, par une gélification et/ou par la formation d'un réseau poreux et au contact de l'eau ou des sucs digestifs, présentent un pouvoir de transformation physique quasi instantané . Ces substances peuvent être utilisées seules et donnent naissance à une gangue visqueuse ou à une structure spongieuse, dans laquelle le ou les principes actifs sont dissous ou dispersés. Ces mêmes substances peuvent être utilisées en association avec d'autres excipients ayant pour but de renforcer la structure de la dite matrice. En association avec d'autres excipients, ces substances matricielles jouent le rôle de « liant ».

On entend par « liant », des substances agissant comme des ciments entre les particules d'un réseau dans le but de renforcer une structure plus ou moins solide.

Ainsi ces substances matricielles évitent la dispersion des autres excipients au sein des sucs digestifs par leur emprisonnement dans la gangue visqueuse ou la structure spongieuse. Cet emprisonnement conduit soit au gonflement progressif soit à la précipitation des excipients au sein même de la dite matrice visqueuse. De ce fait, en fonction de la solidité de la matrice obtenue, la libération d'un principe actif inclus dans un tel système peut varier entre une à vingt quatre heures. Les substances matricielles permettant d'obtenir les dites matrices et jouant le rôle de «liant» appartiennent à la classe des latex inverses. Les latex inverses sont des compositions prêtes à l'emploi largement utilisées dans le domaine pharmaceutique, cosmétique et vétérinaire et qui ont la propriété de se gélifier instantanément au contact de l'eau et des sucs digestifs. Ils sont obtenus à partir d'un mélange contenant:
- une phase huileuse du type
   * huiles minérales : paraffine, isoparaffine et cycloparaffine, etc......
   * huiles minérales blanches : isohexadécane, isododécane, etc....
   * huiles naturelles : hexaméthyltétracosane, squalane, etc....
   * huiles synthétiques : polyisobutène, polyisobuténe hydrogénée, etc....
- une phase aqueuse,
- un tensioactif du type eau dans huile
- un tensioactif du type huile dans eau
- un ou plusieurs monomères du type
   * acrylate
      ∘ acide acrylique, acide métacrylique, acide itaconique, acide maléique, etc......
      ∘ 2 -hydroxyethylmétacrylate, 2,3 -dihydroxypropylacrylate, 2 -hydroxyéthyl métacrylate, 2,3 -dihydroxypropylmétacrylate, et dérivés éthoxylé, etc......
      ° triméthylolpropane triacrylate, éthylène glycol diméthacrylate,
   * acrylamide, acrylamido -2 -methylpropanesulfonate de sodium, méthylènebis(acrylamide), etc......
      - un agent complexant du type acide diallyloxyacétique et sels, triallylamine, diallylurea, etc....

Ces différents mélanges sont soumis à une réaction de polymérisation, suivie d'une étape de distillation.

Les préparations ainsi obtenues se présentent sous forme liquide plus ou moins visqueuse et sont capables d'incorporer des principes actifs soit à l'état liquide soit à l'état solide ainsi que des excipients modulant la biodisponibilité de la préparation ainsi obtenue.

L'incorporation de ces principes actifs lipophiles ou hydrosolubles est favorisée par la présence de tensioactifs au sein de ces latex inverses. Ces tensioactifs permettent aussi de jouer sur la viscosité de la préparation par addition de diluants lipophiles ou hydrophiles.

D'autre part, de telles préparations se prêtent tout à fait à la réalisation de capsules molles ou de gélules "Licaps®" à libération prolongée par la très faible quantité d'eau dans le mélange (distillation). Ces différentes compositions ont fait l'objet d'un certain nombre de brevets sur le plan mondial:
- EP0503853, EP1010708, EP1047716, EP1055707, EP1055451, EP1113029, etc....
- FR2810883, FR2808447, FR2808446, FR2807046, FR2802936, FR2794124, FR2789395, etc...
- WO0135922, WO0032639, etc...
- US2001053801

Certains de ces produits sont commercialisés sous le nom de "SEPIGEL®" et de "SIMULGEL®".

Dans tous les cas, l'étude des différents brevets sur les capsules molles ne fait pas mention de l'utilisation de ces composants comme agent matriciel pour formes à libération prolongée présentées sous forme de capsules molles ou gélules "Licaps®".

Dans le cas des latex inverses, les solvants pouvant être utilisés pour diluer ou dissoudre les agents matriciels sont très variés, de nature lipophiles ou hydrolipophiles permettant ainsi l'incorporation d'un grand nombre de principes actifs dans les dites matrices, que ces derniers soient lipophiles, hydrophiles ou hydrolipophiles.

Les solvants pouvant être utilisés pour diluer ou dissoudre les dits composants présentent des données de toxicité telles qu'elles permettent une utilisation pour le domaine pharmaceutique. Ce sont :
- les huiles végétales, huiles végétales hydrogénées, huiles végétales éthoxylées : huile d'olive, de noisette, de noix de coco, huile de ricin, huile de soja, huile de sésame, etc......
- les huiles minérales : paraffine, isoparaffine, cycloparaffine, huiles de silicones, isohexadécane, isododécane, et dérivés, etc....
- les huiles naturelles, squalane, hexaméthyltétracosane, les mono -, di - et tri -glycérides, etc..
- les huiles synthétiques : polyisobutène, polyisobuténe hydrogénée, etc......
- et autres solvants : éthanol, propanol -1, propanol -2, polypropylène, propylène carbonate, diméthyl isosorbide éther, polyoxyéthylène glycols (Macrogols), glycérol, esters d'acide gras du polyéthylène, esters d'acide gras du propylène glycol, dicaprylate/dicaprate de propylène glycol, caprylate/caprate de glycérol, esters d'acide gras du polyoxyéthylène/polyoxypropylène glycol, triacétin, myristate d'isopropyle, glycofurol, esters liquide d'acide gras, acétate d'éthyle, butanol, propylène glycol acétate, butyl acétate, éthylèneglycol monobutyl éther, éthyle lactate, butyl acétate, éther monoéthylique du diéthylèneglycol, glycérine mono oléate, glycérine linoléate, esters d'acide gras et de glycérol, esters d'acide gras de glycérol et de PEG etc....

La proportion de ces différents solvants utilisés dans ces préparations, dépend de la solubilité des principes actifs et peut varier de 1% à 80% en masse par rapport au poids total des excipients.

Tel que cela a été mis en évidence lors d'un certain nombre d'essais, les latex inverses cités ci-dessus, donnent naissance à un réseau visqueux de consistance molle à consistance ferme ayant l'aspect gélatineux ou à un réseau solide sous forme spongieuse (aspect d'éponge) à structure plus ou moins rigide. Ces structures peuvent être renforcées par introduction, dans le milieu, de substances qui, au contact des sucs digestifs, va accroître la solidité du réseau visqueux ou spongieux. Ceci peut être obtenu :
- par incorporation de substances (sous forme d'adjuvants hydrophiles) présentant un pouvoir de gonflement au contact de l'eau. Emprisonnées aux latex inverses, telles substances gonflent progressivement en ralentissant, de façon non négligeable, la libération du principe actif inclus dans une telle matrice. La consistance de ces dites matrices est proche de celle de la gélatine : structure ferme. Les substances répondant à ce critère appartiennent à la classe :
   * des celluloses et leurs dérivés : méthyl, hydroxypropyl,hydroxyéthyl,hydroxyméthyl, hydroxypropylméthyl, carboxyméthyl, etc...... faiblement substituées ou non, réticulées ou non, et dont les viscosités peuvent aller de 100 mPa.s (cPs) à plus de 100.000 mPa.s (cPs)
   * des amidons et leurs dérivés : amidons de maïs, de pomme de terre, de blé, de riz, de tapioca, natif ou prégélatinisé, ayant subi ou non une dextrénisation, un traitement acide de quelque force que se soit, une oxydation, une réticulation en présence d'acide adipique, acétique, phosphorique, une estérification comme pour les dérivés hydroxypropylés, une éthérification, une transformation enzymatique, etc...... ou une combinaison des dites réactions chimiques citées.
   * des polysaccharides tels que les gommes guar, xanthane, adragante, arabique, caroube, les pectines, les alginates, les carraghénanes, les gellanes, le chitosan, etc......
   * des polymères de la vinylpyrrolidone et ses dérivés.
   Ces substances sont d'autant plus intéressantes que leur pouvoir de gonflement dans l'eau est nettement accru lorsqu'elles sont initialement humidifiées par des solvants organiques, tel est le cas des hydroxypropylméthylcelluloses, des carraghénanes, etc...... La proportion pouvant être introduite dans le milieu pour obtenir une libération comprise entre une heure et vingt quatre heures, est de l'ordre de 0% à 80% en poids par rapport à la masse totale des excipients. Un facteur important pour obtenir une structure compacte et rapide dans le temps, est la taille des particules de ces dites substances. En effet, plus les particules sont fines, plus le pouvoir de gonflement est important et le réseau formé est beaucoup plus dense (moins d'interstices entre les particules). Ainsi, la taille des particules permettant d'obtenir un tel résultat doit être comprise entre 1 *µ*m et 1000 *µ*m avec une préférence pour une taille comprise entre 1 *µ*m et 100 *µ*m.
- par incorporation de plastifiants. Ces substances ont pour but de conférer une certaine élasticité à la matrice de nature spongieuse de manière à contrer les effets néfastes du péristaltisme intestinal sur une structure rigide. Parmi les différentes substances utilisées comme plastifiant, ont été retenus la triacétin, le dibutyl phtalate, le diéthylphtalate, le sébacate de dibutyle et l'isosorbate acétate de saccharose.

Les principes actifs pouvant faire l'objet d'une telle mise en forme appartiennent à toutes les catégories pharmacologiques à savoir les antalgiques, les anti -inflammatoires, les antispasmodiques, les cytotoxiques, les produits cardio-vasculaires (hypertenseurs, hypotenseurs, antiarythmiques, etc....), les antibiotiques, les antifongiques, les antiseptiques, les antiparasitaires, les hormones, les anti-viraux, les antiépileptiques, les antiparkinsoniens, les antimysasthéniques, les migraineux, les antivertigineux, les antiallergiques, les antitussifs, les fluidifiants bronchiques, les analeptiques respiratoires, les neuroleptiques, les anxiolytiques, les hypnotiques, les antidépresseurs, les normothymiques, les psychostimulants, les sédatifs, les myorelaxants, les diurétiques, etc......

Différents exemples sont donnés ci-après illustrant les différentes possibilités d'obtenir des systèmes matriciels à libération prolongée encapsulés.

Ces principes actifs peuvent être incorporés :
- sous forme liquide : solutions hydro-lipophiles, émulsions, micro-émulsions auto-dispersibles, etc......
- sous forme solide à l'état de :
   * poudres présentant une répartition granulométrique allant du micron à 1000 *µ*m
   * microgranules ou pellets enrobés ou non ayant une granulométrie allant de 10*µ*m à 1000 *µ*m
   * absorbats : produits liquides fixés sur support neutre pour accroître la stabilité du principe actif, la granulométrique de ces supports allant du micron à 1000 *µ*m.

Les différents solvants pouvant être utilisés pour solubiliser ou disperser ces différents principes actifs sont identiques à ceux décrits précédemment pour la dilution des latex inverses.

A ces différents solvants, peuvent être ajoutés des tensioactifs facilitant la dispersion ou la solubilisation des principes actifs. Les tensioactifs pouvant être utilisés dans la présente invention sont :
- les tensioactifs non ioniques :
   * des esters de sorbitane : polysorbates, spans, tweens, etc...
   * des acides gras polyéthoxylés : stéarate de PEG 8 au stéarate de PEG 100;
   * des alcools gras polyéthoxylés : mélange d'éther de monolaurate de PEG ayant de 4 à 23 groupes oxyéthylènés sur la chaîne polyoxyéthylénique, etc...
   * des esters de glycol : stéarate de méthylglycol;
   * des esters de glycérol : monostéarate de glycérol, stéarate de PEG 75, stéarate de glycol et de PEG 6 -32, etc...
   * des esters de PEG;
   * des esters de saccharose;
   * des éthers d'alcool gras et de PEG : Brij;
   * des éthers d'alkyl phénol et de PEG;
   * des tensioactifs présentant une fonction amide :
      ∘ monoéthanolamide d'acide gras de coprah, d'acide laurique, etc...
      ∘ diéthanolamide d'acide myristique, d'acide laurique, etc...
      ∘ mono -isopropanolamine d'acide laurique.
   * les lécithines
- les tensioactifs ioniques :
   * des dérivés sulfatés : le laurylsulfate de sodium et ses dérivés;
   * des dérivés sulfonés : dodécylsulfosuccinate de sodium et ses dérivés;
   * des ammoniums quaternaires: chlorure de cétyltriméthylammonium, laurylpyridinium, distéaryldiméthylammonium, etc...

- amphotères : bétaïne d'ammonium d'alkyldiméthyle de coprah, dérivés d'amides d'acide gras à structure bétaïnique, acide lauryl-a-iminodipropionique et ses dérivés, acide lauryl-myristyl-a-aminopropionique et ses dérivés, etc...

La quantité de ces substances utilisée pour favoriser la solubilisation ou la dispersion des principes actifs peut varier de 0 à 50% en poids par rapport à la masse totale des excipients.

En fonction du type de matrice obtenue au contact des sucs digestifs, des accélérateurs de dissolution peuvent être incorporés dans le mélange final. Ces substances ont pour but, par leur dissolution rapide au contact des sucs digestifs, de créer un réseau poreux si celui obtenu avec le système matriciel est trop compact. Ces substances sont mises en suspension dans le milieu lipophile. Ce sont :
- le lactose,
- les phosphates mono et basiques (calcium, sodium, potassium),
- les carbonates (calcium, sodium, potassium)
- les polyols : sorbitol, maltodextrines, dextrose, maltitol, xylitol, maltisorb, manitol, etc......

Ces substances sont utilisées à une concentration pouvant varier de 0 à 50% en poids par rapport à la masse totale des excipients.

Dans certains cas, il est nécessaire de faire appel à des systèmes tampons pour maintenir en suspension ou permettre la solubilisation des principes actifs et voire même augmenter la viscosité intrinsèque de la matrice si cette dernière est sensible à l'influence du pH du milieu de dissolution. Les composants permettant d'obtenir ces objectifs sont des acides et des bases et leurs sels correspondants. Ainsi, peuvent être utilisés :
- l'acide citrique et les sels sodiques, potassiques et calciques
- l'acide phosphorique (ortho et meta) et les sels sodiques, potassiques et calciques, mono et dibasiques
- les carbonates sodiques, potassiques et calciques
- l'acide phtalique et les sels sodiques, potassiques et calciques
- l'acide chlorhydrique et les sels sodiques, potassiques et calciques
- l'acide borique et les sels sodiques, potassiques et calciques
- l'acide acétique et les sels sodiques, potassiques et calciques
- l'acide lactique et les sels sodiques, potassiques et calciques
- l'acide propionique et les sels sodiques, potassiques et calciques
- les hydroxydes de sodium, potassium et calcium

La proportion de ces différents adjuvants utilisés seuls ou en combinaison, et variant en fonction du but recherché, est comprise entre 0% et 50% en poids par rapport à la masse totale des excipients. Ces substances peuvent être introduites sous forme dissoute ou à l'état solide dans la préparation. A l'état solide, ces différents composants forment un micro environnement basique ou acide intra-matriciel lors de leur solubilisation progressive permettant ainsi de moduler la libération du principe à partir de ce système.

Les solutions ou suspensions ainsi réalisées donnant naissances in situ à des systèmes matriciels à libération prolongée, présentent des viscosités allant de 50 millipascales et 500.000 millipascales. Ces solutions ou suspensions sont conditionnées en capsules molles ou en gélules types "Licaps®".

La paroi de ces gélules ou capsules molles peuvent être constituées de gélatine mais aussi de carraghénanes, d'amidons ou d'hydroxypropylméthyl cellulose.

De tels systèmes, après ouverture ou dissolution de la tunique, permettent la libération progressive du principe actif sur une période pouvant aller de une heure à vingt quatre heures, cette cinétique de libération étant peu ou pas dépendante des facteurs biologiques environnants. La cinétique de dissolution, fonction ou non du pH, peut être d'ordre zéro ou un, selon le type d'excipients utilisés pour obtenir une telle libération.

L'invention n'est pas limitée dans son application car les principes actifs peuvent appartenir à toutes les classes thérapeutiques.

La présente invention porte également sur le procédé de fabrication des dites compositions selon la revendication 23 par un simple mélange à froid ou à chaud des différents constituants suivi d'un conditionnement en capsules molles ou gélules. Réalisée à chaud ou à froid selon les composants de la formule mis en oeuvre, la fabrication comporte deux ou trois étapes principales selon l'agent matriciel mis en oeuvre. Les exemples de réalisation figurant ci -après sont des formules possibles de compositions selon la présente invention qu'ils ne limitent en aucune façon.

### Exemple 1 :

### Ibuprofène : capsules molles à libération prolongée

- Ibuprofène* .............................. 200,0000 g
- Latex inverse ("Sepigel 305®")QS........ 600,0000 g

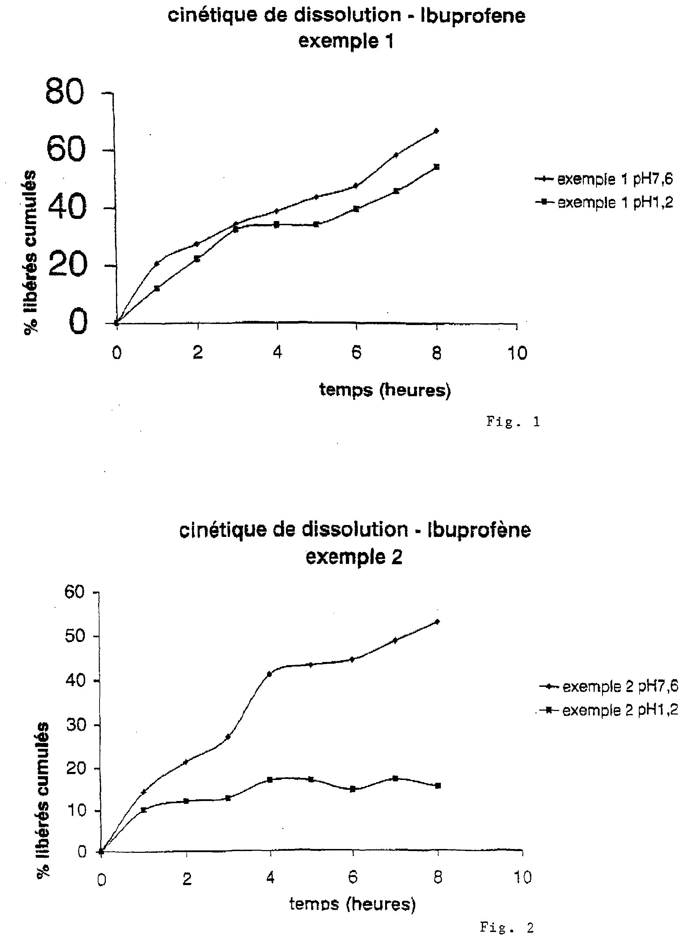

Dans un bécher de 2 litres, introduire le "Sepigel 305®". Ajouter l'ibuprofène. Homogénéiser pendant 30 minutes jusqu'à obtention d'un mélange homogène. Conditionner en capsules molles 8 oblongs. L'étude de la cinétique de dissolution à pH 7.6 montre que la libération du principe est supérieure à 8 heures et à 2 heures à pH 1.2 (cf.fig. 1). L'ibuprofène ("Basf") utilisée présente une granulométrie moyenne de 25 *µ*m.

### Exemple 2 :

### Ibuprofène : capsules molles à libération prolongée

- Ibuprofène................................ 200,0000 g
- Hydroxypropyl amidon...................... 200,0000 g
- Latex inverse ("Sepigel 305®")QS............. 600,0000 g

Mélanger intimement l'ibuprofène avec l'hydroxypropyl amidon. Dans un bécher de 2 litres, introduire le "Sepigel 305®". Ajouter le mélange ibuprofène/hydroxypropyl amidon. Homogénéiser pendant 30 minutes jusqu'à obtention d'un mélange homogène. Conditionner en capsules molles 8 oblongs. L'étude de la cinétique de dissolution à pH 7.6 montre que la libération du principe est supérieure à 8 heures et à 2 heures à pH 1.2 (cf. fig. 2).

### Exemple 3 :

### Ibuprofène : capsules molles à libération prolongée

- Ibuprofène................................ 200,0000 g
- "Montane 20®"........................ 400,0000 g
- Latex inverse ("Sepigel 305®")QS................ 1100,0000 g

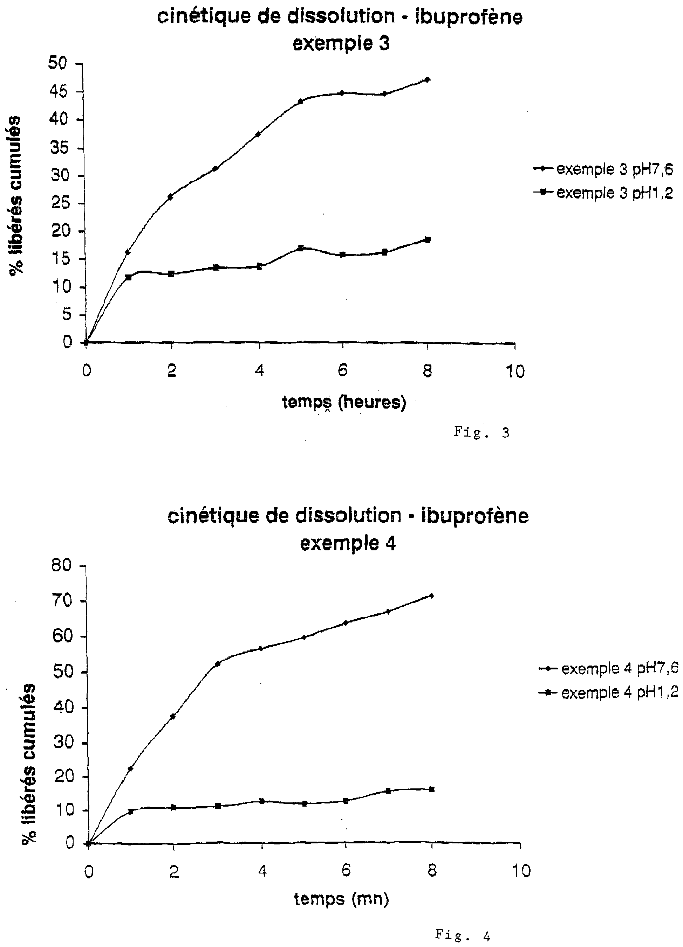

Chauffer à 40°C le mélange ibuprofène/"Montane 20®". Dans un bécher de 2 litres, introduire le "Sepigel 305®". Ajouter le mélange ibuprofène/"Montane 20®". Homogénéiser pendant 30 minutes jusqu'à obtention d'un mélange homogène. Conditionner en capsules molles 8 oblongs. L'étude de la cinétique de dissolution à pH 7.6 montre que la libération du principe est supérieure à 8 heure et à 2 heures à pH 1.2 (cf. fig. 3).

### Exemple 4 :

### Ibuprofène : capsules molles à libération prolongée

- Ibuprofène................................ 200,0000 g
- Glycérine mono-oléate..................... 400,0000 g
- Latex inverse ("Sepigel 305®")QS.......... 1100,0000 g

Chauffer à 40°C le mélange ibuprofène/Glycérine monooléate.Dans un bécher de 2 litres introduire le "Sepigel 305®". Ajouter le mélange ibuprofène/Glycérine mono-oléate. Homogénéiser pendant 30 minutes jusqu'à obtention d'un mélange homogène. Conditionner en capsules molles 8 oblongs. L'étude de la cinétique de dissolution à pH 7.6 montre que la libération du principe est supérieure à 8 heures et à 2 heures à pH 1.2 (cf.fig. 4).

### Exemple 5 :

### Ibuprofène : capsules molles à libération prolongée

- Ibuprofène*............................. 200,0000 g
- Phosphate potassique monobasique........ 6,8050 g
- Hydroxyde de sodium.................... 0,0848 g
- Latex inverse ("Sepigel 305®") QS....... 1000,0000 g Dans un bécher de 2 litres introduire le "Sepigel 305®".

Ajouter l'ibuprofène, l'hydroxyde de sodium et le phosphate monobasique de potassium. Homogénéiser pendant 30 minutes jusqu'à obtention d'un mélange homogène. Conditionner en capsules molles 8 oblongs. L'étude de la cinétique de dissolution à pH 7.6 montre que la libération du principe est supérieure à 8 heures et à 2 heures à pH 1.2. - l'ibuprofène ("Basf") utilisée présente une granulométrie moyenne de 25 *µ*m.

### Exemple 6 :

### Ibuprofène : capsules molles à libération prolongée

- Ibuprofène............................. 200,0000 g
- Phosphate monosodique................... 1,3000 g
- Phosphate disodique.................... 24,4000 g
- Latex inverse ("Sepigel 305®")QS........... 1000,0000 g

Dans un bécher de 2 litres introduire le "Sepigel 305®". Ajouter l'ibuprofène, le phosphate monosodique et le phosphate disodique. Homogénéiser pendant 30 minutes jusqu'à obtention d'un mélange homogène. Conditionner en capsules molles 8 oblongs. L'étude de la cinétique de dissolution à pH 7.6 montre que la libération du principe est supérieure à 8 heures et à 2 heures à pH 1.2.

### Exemple 7:

### Paracétamol capsules molles à libération prolongée

- Paracétamol.............................. 100,0000 g
- Glycérine linoléate*................... 450,0000 g
- Copolymère neutre de l'acide métacrylique** 100,0000 g
- "Sepigel 305®"***...................................... 350,0000 g

Dissoudre à 100°C le copolymère neutre de l'acide métacrylique dans le linoléate de glycérine. Laisser refroidir à 30°C -35°C et ajouter le paracétamol. Homogénéiser. Ajouter le Sepigel 305®. Homogénéiser pendant 30 minutes. Conditionner en capsules molles 8 oblongs. L'étude de la cinétique de dissolution à pH 7.6 montre que la libération du principe est supérieure à 8 heures et à 2 heures à pH 1.2.
* : "Maisine® : Gattefossé"
** : "Plastoid B® : Röhm Pharma"
*** : "Sepigel 305® : Seppic"

### Exemple 8:

### Diclofénac capsules molles à libération prolongée

- Diclofénac.............................. 25,0000 g
- Ether monoéthylique du diéthylène glycol*. 450,0000 g
- Hydroxypropylcellulose faiblement substitué**............................... 25,0000 g
- "Sepigel 305®"***................... 50,00000 g

Dissoudre à 70°C l'hydroxypropylcellulose faiblement substitué dans l'éther monoéthylique du diéthylèneglycol. Laisser refroidir à 25°C et ajouter le diclofénac. Homogénéiser. Ajouter le Sepigel 305®. Homogénéiser pendant 30 minutes. Conditionner en capsules molles 4 oblongs. L'étude de la cinétique de dissolution à pH 7.6 montre que la libération du principe est supérieure à 8 heures et à 2 heures à pH 1.2.
* : "Transcutol P ® : Gattefossé"
** : "HP 55® : Seppic"
*** : "Sepigel 305® : Seppic"

### Exemple 9:

### Dimenhydrinate capsules molles à libération prolongée

- Dimenhydrinate............................ 50,0000 g
- Ether monoéthylique du diéthylène glycol*. 425,0000 g
- "Sepigel 305®"............................ 400,0000 g
- Sucrose acétate isobutyrate............... 25,0000 g Dissoudre le sucrose acétate isobutyrate dans l'éther monoéthylique du diéthylèneglycol. Ajouter le dimenhydrinate. Homogénéiser pendant 10 minutes. Ajouter l'hydroxypropyl amidon. Homogénéiser pendant 10 minutes. Ajouter le "Sepigel 305®". Homogénéiser pendant 30 minutes. Conditionner en capsules molles 8 oblongs. L'étude de la cinétique de dissolution à pH 7.6 montre que la libération du principe est supérieure à 8 heures.
   * : "Transcutol P ® : Gattefossé"

## Revendications

1. Compositions liquides sous forme de capsules à libération prolongée destinées à la voie orale, **CARACTÉRISÉES PAR LE FAIT Qu'**elles comprennent :
(i) au moins un agent matriciel liquide appartenant à la famille des latex inverses constitués par des dérivés de l'acide acrylique ou par des polymères d'acrylamides
(ii) au moins un principe actif
(iii) un solvant de solubilisation ou de dispersion du principe actif
(iv) et au moins un excipient modulant la cinétique de libération du principe actif
les susdits excipients modulant la libération étant composés:
a) d'adjuvants hydrophiles appartenant à la classe des celluloses et leur dérivés, des amidons et leur dérivés, des polysaccharides telles que les gommes guar, xanthane, adragante, arabique, caroube, les pectines, les alginates, les carraghénanes, les gellanes, le chitosan, des polymères de la vinylpyrrolidone,
b) de plastifiants,
c) de tensioactifs,
d) d'accélérateurs de dissolution,
e) de systèmes tampons,
ou l'association des cinq,
conduisant à une libération prolongée du principe actif sur une période comprise entre 1 à 24 heures par formation in situ d'un réseau visqueux de consistance molle à consistance ferme ayant l'aspect gélatineux ou d'un réseau solide sous forme spongieuse (aspect d'éponge) à structure plus ou moins rigide , par modification physique instantanée dudit agent matriciel liquide au contact des sucs digestifs dès ouverture de la capsule.

2. Compositions liquides selon la revendication 1, **CARACTÉRISÉES PAR LE FAIT QUE** le principe actif est à l'état liquide ou à l'état solide.

3. Compositions liquides selon la revendication 1, **CARACTÉRISÉES PAR LE FAIT QUE** la modification physique instantanée du contenu de la capsule est obtenue par une gélification et/ou par la formation d'un réseau poreux des latex inverses au contact des sucs digestifs.

4. Compositions liquides selon la revendication 1, **CARACTÉRISÉES PAR LE FAIT QUE** la cinétique de dissolution du principe actif à partir de la matrice est fonction ou non du pH.

5. Compositions liquides selon la revendication 1, **CARACTÉRISÉES PAR LE FAIT QUE** la cinétique de dissolution du principe actif à partir de la matrice est fonction des enzymes digestives.

6. Compositions liquides selon la revendication 1, **CARACTÉRISÉES PAR LE FAIT QUE** le principe actif est dissout ou dispersé dans des huiles ou des solvants organiques à caractère lipophile, hydrophile ou hydro-lipophile.

7. Compositions liquides selon la revendication 1, **CARACTÉRISÉES PAR LE FAIT QU'**elles sont conditionnées dans une capsule dure ou molle.

8. Compositions liquides selon la revendication 1, **CARACTÉRISÉES PAR LE FAIT QUE** la composition de la tunique de la capsule est constituée de gélatine, ou d'amidons, ou d'hydroxypropylméthylcelluloses, ou de carraghénanes ou de polymères de l'alcool polyvinylique.

9. Compositions liquides selon la revendication 1, **CARACTÉRISÉES PAR LE FAIT QUE** le susdit principe actif appartient à toutes les classes thérapeutiques.

10. Compositions liquides selon la revendication 2, **CARACTÉRISÉES PAR LE FAIT QUE** le principe actif à l'état liquide est incorporé sous forme d'une solution, d'une émulsion ou d'une microémulsion auto-dispersible.

11. Compositions liquides selon la revendication 2, **CARACTÉRISÉES PAR LE FAIT QUE** le principe actif à l'état solide est dispersé sous forme de poudre enrobée ou non, ou d'absorbats de titre connu.

12. Compositions liquides selon la revendication 2, **CARACTÉRISÉES PAR LE FAIT QUE** le principe actif dispersé à l'état solide présente une granulométrie comprise entre 1 *µ*m et 1000 *µ*m.

13. Compositions liquides selon la revendication 1, **CARACTÉRISÉES PAR LE FAIT QUE** la concentration en adjuvants hydrophiles modulant la libération est comprise entre 0% à 80% en poids par rapport à la masse totale des excipients.

14. Compositions liquides selon la revendication 1, **CARACTÉRISÉES PAR LE FAIT QUE** la granulométrie des adjuvants hydrophiles doit être comprise entre 1 *µ*m et 1000 *µ*m.

15. Compositions liquides selon la revendication 1, **CARACTÉRISÉES PAR LE FAIT QUE** les plastifiants sont constitués par la triacétine, le dibutyle phtalate, le diéthyle phtalate, le sébacate de dibutyle et l'isobutyrate acétate de saccharose.

16. Compositions liquides selon la revendication 1, **CARACTÉRISÉES PAR LE FAIT QUE** la concentration en plastifiant est comprise entre 0% à 80% en poids par rapport à la masse totale des excipients.

17. Compositions liquides selon la revendication 1, **CARACTÉRISÉES PAR LE FAIT QUE** les susdits agents tensioactifs appartiennent à la classe des tensioactifs ioniques, non ioniques et amphotères.

18. Compositions liquides selon la revendication 1, **CARACTÉRISÉES PAR LE FAIT QUE** la concentration en tensioactifs est comprise entre 0 et 50% en masse par rapport à la masse totale des excipients.

19. Compositions liquides selon la revendication 1, **CARACTÉRISÉES PAR LE FAIT QUE** les susdits accélérateurs de dissolution sont constitués par le lactose, ou les polyols dont le sorbitol, le maltitol, le xylitol, les maltodextrines, le maltisorb, le manitol, ou les carbonates et les phosphates mono et dibasiques.

20. Compositions liquides selon la revendication 1, **CARACTÉRISÉES PAR LE FAIT QUE** la concentration en accélérateurs de dissolution est comprise entre 0 et 50% en masse par rapport à la masse totale des excipients.

21. Compositions liquides selon la revendication 1, **CARACTÉRISÉES PAR LE FAIT QUE** les susdits systèmes tampons sont constitués par les acides chlorhydrique, phtalique, borique, citrique, phosphorique, acétique, lactique, propionique et les sels correspondants et les hydroxydes de sodium, calcium et potassium.

22. Compositions liquides selon la revendication 1, **CARACTÉRISÉES PAR LE FAIT QUE** la concentration en systèmes tampon est comprise entre 0% et 50% en masse par rapport à la masse totale des excipients.

23. Procédé de fabrication de compositions liquides selon l'une quelconque des revendications 1 à 22, **CARACTÉRISÉ EN CE QUE** les différents constituants de ces dites compositions liquides sont mélangés à froid ou à chaud et suivis d'un conditionnement en capsules molles ou en gélules.

## Patentansprüche

1. Flüssige Zusammensetzungen in Form von Kapseln mit verlängerter Freisetzung, die für den oralen Weg bestimmt sind, **dadurch gekennzeichnet, dass** sie umfassen:
(i) mindestens ein flüssiges Matrixmittel, das zur Gruppe der Inverslatexe gehört, die durch Derivate der Acrylsäure oder durch Acrylamid-Polymere gebildet werden
(ii) mindestens einen Wirkstoff
(iii) ein Lösungsmittel zur Solubilisierung oder Dispersion des Wirkstoffes
(iv) und mindestens einen Hilfsstoff, der die Freisetzungskinetik des Wirkstoffes moduliert
wobei sich die zuvor genannten Hilfsstoffe, die die Freisetzung modulieren, zusammensetzen:
a) aus hydrophilen Zusatzmitteln, die der Klasse der Cellulosen und deren Derivaten, den Stärken und deren Derivaten, den Polysacchariden, wie den Guar-, Xanthan-, Tragant-, Arabicum-, Johannisbrot-Gummi, den Pektinen, den Alignaten, Carrageen, den Gellanen, Chitosan, Polymeren von Vinylpyrrolidon angehören,
b) aus Weichmachern,
c) aus Tensiden,
d) aus Auflösungsbeschleunigern,
e) aus Puffersystemen,
oder der Verbindung der fünf,
zu einer verlängerten Freisetzung des Wirkstoffes innerhalb eines Zeitraums führend, der zwischen 1 und 24 Stunden enthalten ist, durch die Bildung vor Ort eines zähflüssigen Netzwerks mit einer weichen Konsistenz bis zu einer festen Konsistenz, die ein gallertartiges Aussehen aufweist, oder eines festen Netzwerks in einer schwammigen Form (Aussehen eines Schwamms) mit einer mehr oder weniger starren Struktur, durch eine augenblickliche physikalische Veränderung des besagten flüssigen Matrixmittels bei Kontakt mit den Verdauungssäften unmittelbar nach dem Öffnen der Kapsel.

2. Flüssige Zusammensetzungen nach Anspruch 1, **dadurch gekennzeichnet, dass** der Wirkstoff im flüssigen Zustand oder im festen Zustand ist.

3. Flüssige Zusammensetzungen nach Anspruch 1, **dadurch gekennzeichnet, dass** man die augenblickliche physikalische Veränderung des Inhalts der Kapsel durch Gelierung und/oder durch die Bildung eines porösen Netzwerks der Inverslatexe in Kontakt mit den Verdauungssäften erhält.

4. Flüssige Zusammensetzungen nach Anspruch 1, **dadurch gekennzeichnet, dass** die Auflösungskinetik des Wirkstoffs aus der Matrix vom ph-Wert abhängig ist oder nicht.

5. Flüssige Zusammensetzungen nach Anspruch 1, **dadurch gekennzeichnet, dass** die Auflösungskinetik des Wirkstoffs aus der Matrix von den Verdauungsenzymen abhängig ist.

6. Flüssige Zusammensetzungen nach Anspruch 1, **dadurch gekennzeichnet, dass** der Wirkstoff in Ölen oder organischen Lösungsmitteln mit lipophilem, hydrophilem oder hydro-lipophilem Charakter aufgelöst oder verteilt wird.

7. Flüssige Zusammensetzungen nach Anspruch 1, **dadurch gekennzeichnet, dass** sie in einer harten oder weichen Kapsel konditioniert werden.

8. Flüssige Zusammensetzungen nach Anspruch 1, **dadurch gekennzeichnet, dass** die Zusammensetzung des Mantels der Kapsel aus Gelatine oder Stärken oder Hydropropylmethylcellulosen, oder Carrageen oder aus Polymeren des Polyvinylalkohols gebildet wird.

9. Flüssige Zusammensetzungen nach Anspruch 1, **dadurch gekennzeichnet, dass** der zuvor genannte Wirkstoff zu allen therapeutischen Klassen gehört.

10. Flüssige Zusammensetzungen nach Anspruch 2, **dadurch gekennzeichnet, dass** der Wirkstoff im flüssigen Zustand in Form einer Lösung, einer Emulsion oder einer selbstauflösenden Mikroemulsion integriert wird.

11. Flüssige Zusammensetzungen nach Anspruch 2, **dadurch gekennzeichnet, dass** der Wirkstoff im festen Zustand in Form von überzogenem oder nicht überzogenem Pulver oder von Absorbaten bekannten Gehalts verteilt wird.

12. Flüssige Zusammensetzungen nach Anspruch 2, **dadurch gekennzeichnet, dass** der Wirkstoff im festen Zustand eine Korngröße zwischen 1 µm und 1000 µm aufweist.

13. Flüssige Zusammensetzungen nach Anspruch 1, **dadurch gekennzeichnet, dass** die Konzentration an hydrophilen Zusatzmitteln, die die Freisetzung modulieren, zwischen 0% bis 80% an Gewicht im Verhältnis zur Gesamtmasse der Hilfsstoffe enthalten ist.

14. Flüssige Zusammensetzungen nach Anspruch 1, **dadurch gekennzeichnet, dass** die Korngröße der hydrophilen Zusatzmittel zwischen 1 µm und 1000 µm enthalten sein muss.

15. Flüssige Zusammensetzungen nach Anspruch 1, **dadurch gekennzeichnet, dass** die Weichmacher durch Triacetin, Dibutylphtalat, Diethylphtalat, Dibutylsebacat und Saccharoseacetatisobutyrat gebildet werden.

16. Flüssige Zusammensetzungen nach Anspruch 1, **dadurch gekennzeichnet, dass** die Weichmacherkonzentration zwischen 0% bis 80% an Gewicht im Verhältnis zur Gesamtmasse der Hilfsstoffe enthalten ist.

17. Flüssige Zusammensetzungen nach Anspruch 1, **dadurch gekennzeichnet, dass** die zuvor genannten Tenside zur Klasse der ionischen, nicht ionischen und amphoteren Tenside gehören.

18. Flüssige Zusammensetzungen nach Anspruch 1, **dadurch gekennzeichnet, dass** die Tensidkonzentration zwischen 0 und 50% an Masse im Verhältnis zur Gesamtmasse der Hilfsstoffe enthalten ist.

19. Flüssige Zusammensetzungen nach Anspruch 1, **dadurch gekennzeichnet, dass** die zuvor genannten Auflösungsbeschleuniger durch Lactose, oder Polyole, darunter Sorbitol, Malitol, Xylitol, Maltodextrine, Maltisorb, Manitol oder Karbonate und ein- unnd zweibasige Phosphate gebildet werden.

20. Flüssige Zusammensetzungen nach Anspruch 1, **dadurch gekennzeichnet, dass** die Konzentration an Auflösungsbeschleunigern zwischen 0 und 50% an Masse im Verhältnis zur Gesamtmasse der Hilfsstoffe enthalten ist.

21. Flüssige Zusammensetzungen nach Anspruch 1, **dadurch gekennzeichnet, dass** die zuvor genannten Puffersysteme aus Salz-, Phthal-, Bor-, Zitronen-, Phosphor-, Essig-, Milch-, Propionsäure und die entsprechenden Salze und Natrium-, Kalzium und Kaliumhydroxid gebildet werden.

22. Flüssige Zusammensetzungen nach Anspruch 1, **dadurch gekennzeichnet, dass** die Konzentration an Puffersystemen zwischen 0% und 50% an Masse im Verhältnis zur Gesamtmasse der Hilfsstoffe enthalten ist.

23. Verfahren zur Herstellung von flüssigen Zusammensetzungen nach einem der Ansprüche 1 bis 22, **dadurch gekennzeichnet, dass** die verschiedenen Bestandteile dieser besagten flüssigen Zusammensetzungen kalt oder warm gemischt werden, und gefolgt von einer Konditionierung in weichen Kapseln oder in Hartkapseln.

## Claims

1. Liquid compositions under the form of sustained release caspsules intended for oral administration, wherein they comprise :
(i) at least one liquid matricial agent belonging to the family of inverted latexes constituted of derivatives of acrylic acid or of acrylamide polymers
(ii) at least one active substance
(iii) one solubilization or dispersion solvent of the active substance
(iv) and at least one excipient modulating the release kinetics of the active substance
the abovementioned excipients modulating the release being composed of :
a) hydrophilic additives belonging to the class of celluloses and their derivatives, of starches and their derivatives, of polysaccharides such as guar, xanthan, tragacanth, and acacia gums, carob, pectins, alginates, carraghenanes, gellan gums, chitosan, polymers of vinylpyrrolidone,
b) plasticizers,
c) tensioactives,
d) dissolution accelerators,
e) buffer systems,
or of the association of the five,
leading to a sustained release of the active substance through a period ranging from one to twenty-four hours by the in situ formation of a viscous lattice with a soft to hard consistency having a gelatinous aspect or to a solid lattice under poriferous form (aspect of a sponge) with a more or less rigid structure, by means of an instantaneous and physical modification of said liquid matricial agent at the contact of digestive secretions as soon as the capsule is opened.

2. Liquid compositions according to claim 1, wherein the active substance is in the liquid state or in the solid state.

3. Liquid compositions according to claim 1, wherein the instantaneous physical modification of the content of the capsule is obtained by means of a gelification and/or by the formation of a porous lattice of inverted latexes at the contact of digestive secretions.

4. Liquid compositions according to claim 1, wherein the dissolution kinetics of the active substance from the matrice is a function or not of the pH.

5. Liquid compositions according to claim 1, wherein the dissolution kinetics of the active substance from the matrice is a function of the digestive enzymes.

6. Liquid compositions according to claim 1, wherein the active substance is dissolved or dispersed into oils or organic solvents of lipophilic, hydrophilic or hydrolipophilic nature.

7. Liquid compositions according to claim 1, wherein they are conditioned in a hard or soft capsule.

8. Liquid compositions according to claim 1, wherein the composition of the tunic of the capsule is constituted of gelatine or starches or hydroxypropylmethylcelluloses or carraghenanes or of polymers of polyvinylic alcohol.

9. Liquid compositions according to claim 1, wherein the abovementioned active substance belongs to all therapeutical classes.

10. Liquid compositions according to claim 2, wherein the active substance in the liquid state is incorporated under the form of a solution, an emulsion or an auto-dispersible micro-emulsion.

11. Liquid compositions according to claim 2, wherein the active substance in the solid state is dispersed under the form of a powder, which may be coated or not, or under the form of absorbats of known title.

12. Liquid compositions according to claim 2, wherein the active substance dispersed in the solid state shows a granulometry comprised between 1 µm and 1000 µm.

13. Liquid compositions according to claim 1, wherein the concentration of hydrophilic additives modulating the release is comprised between 0 % and 80 % in weight with respect to the total mass of the excipients.

14. Liquid compositions according to claim 1, wherein the granulometry of the hydrophilic additives must be comprised between 1 µm and 1000 µm.

15. Liquid compositions according to claim 1, wherein the plasticizers are constituted of triacetin, dibutyl phthalate, diethyl phthalate, dibutyle sebacate and saccharose isobutyrate acetate.

16. Liquid compositions according to claim 1, wherein the concentration in plasticizer is comprised between 0 % and 80 % in weight with respect to the total mass of the excipients.

17. Liquid compositions according to claim 1, wherein the abovementioned tensioactive agents belong to the class of ionic, non ionic and amphoteric tensioactives.

18. Liquid compositions according to claim 1, wherein the concentration of tensioactives is comprised between 0 % and 50 % in mass with respect to the total mass of the excipients.

19. Liquid compositions according to claim 1, wherein the abovementioned dissolution accelerators are constituted of lactose or polyols, including sorbitol, maltitol, xylitol, maltodextrines, maltisorb, manitol or carbonates and the mono and dibasic phosphates.

20. Liquid compositions according to claim 1, wherein the concentration of dissolution accelerators is comprised between 0 % and 50 % in mass with respect to the total mass of the excipients.

21. Liquid compositions according to claim 1, wherein the abovementioned buffer systems are constituted of hydrochloric, phthalic, boric, citric, phosphoric, acetic, lactic, propionic acids and the corresponding salts and the sodium, calcium and potassium hydroxides.

22. Liquid compositions according to claim 1, wherein the concentration of buffer systems is comprised between 0 % and 50 % in mass with respect to the total mass of the excipients.

23. Manufacturing method of liquid compositions according to any one of claims 1 to 22, wherein the different components of said liquid compositions are mixed with or without heat and are followed by a conditioning in soft capsules or in hard capsules.
